# EUROPEAN PATENT APPLICATION

(11) **EP 3 409 218 A1**
(43) Date of publication of application: **05.12.2018**
(21) Application number: 17173594.7
(22) Date of filing: 30.05.2017
(51) Int. Cl.: A61B 17/15

(54) **JIG FOR USE IN KNEE REPLACEMENT SURGERY**

(71) Applicant: Samo S.p.A., 40057 Bologna (IT)
(72) Inventor: The designation of the inventor has not yet been filed
(74) Representative: Dean, John Paul

(57) **Abstract**

The invention provides a cutting, trialling and sizing jig for use in knee replacement surgery according to claim 1.

## Description

### Field of the invention

This invention relates to the field of orthopaedic surgery. In particular, the invention relates to a kit for knee replacement surgery, to components and tools of the kit, and to methods of knee replacement using the kit, components and tools. More particularly, the invention relates to the field of secondary knee replacement surgery, which is also known as corrective, or revisionary, knee replacement surgery.

### Background

Knee replacement surgery, where implants are fixed to the modified opposing ends of a patient's femur and tibia to replace worn joint surfaces, and to provide a functioning, artificial joint, is now a relatively routine surgical procedure. Conventional knee replacement surgery involves a process of trial and error in fitting a series of different sized and shaped trial implants, which vary according to the patient's physiology, assessing how the trial implants function when the artificial knee joint is in flexion and in extension, before equivalent final replacement implants are fitted. Surgeons are provided with kits of components, including several trial implants of different sizes, and combinations of shims and spacers, and tools for each procedure. This methodology has several disadvantages in practice. First, large numbers of components and tools, are required by conventional kits, many of which will not be used in the surgical procedure but are still required to be, stored, handled and sterilised. Most of those unused components are the differently sized trial implants. Second, the procedures are physical and time consuming.

A critical aspect for success in knee replacement surgery is restoring the joint line, i.e. arranging the femoral and tibial implants in relation to the remaining femur and tibia so that they articulate around substantially the same axis as for the un-operated knee joint, as joint line position plays an important role in knee movement and function. Joint line position is determined in terms of the anterior-posterior size of the unoperated femur, the rearward offset of the tibial implant, and through arranging the tibial implant to cover as much of the upper surface of the operated tibia. Studies have shown that restoration of the joint line leads to improved knee function, better ligament stability, and an improved range of movement.

Another important aspect of knee replacement surgery is ligament balancing i.e. arranging the operation of the replacement joint so that any remaining lateral and medial collateral ligaments on either side of the knee are balanced, and so not unevenly strained, during operation of the knee joint - as this would lead to ligamentous instability.

The correct location of the patella, whether natural or artificial, is also important in knee replacement surgery.

Some patients may need a more complex type of knee replacement known as secondary knee replacement, where there has been significant bone loss due to arthritis or a fracture, a major deformity of the knee, or where there is weakness of the main knee ligaments leading to joint instability. Such revision knee replacements are performed in cases of second or third replacements in a particular knee. The surgical procedure for revision knee replacement surgery is more complicated, as the surgeon has less bone to work with, as landmarks on the femur and tibia are lost as a result of the primary knee replacement, and because of the need to fit the implants so as to restore the joint line as well as possible. In practice, elevation of the joint line is common in revision knee replacement. An increase in joint line elevation of only 8mm or more leads to reduced knee function.

The femoral and tibial implants for secondary knee replacements usually have a long stem, which is securely fixed into the bone cavity (diapyhseal canal), allowing the implant components greater stability. The implant components may also interlock in the centre of the knee to form a hinge also to give greater stability. Extra pieces of metal and/or plastic may be used to compensate for any removed or badly damaged bone.

Conventionally, the components and tools for secondary knee replacement surgery are supplied to a surgeon in a set of 8 to 20 instrument boxes weighing a total of 100 to 200kg. Sterilising such a large amount of surgical componentry is expensive - typically costing €1-2000 per operation simply to sterilise. All those boxes and the components and tools that they contain must be sterilised before use, leading to unnecessary costs.

More recently, secondary knee replacement kits have been offered which include only two instrument boxes. However, those kits still involve a large number of trial implants, and do not involve innovation in the measuring, cutting or trialling functions.

There is a need for simplification of the kits for knee replacement surgery in order to reduce sterilisation, handling and storage costs. There is also a need to reduce the time taken for surgery, in order to reduce the impact on patients and to improve surgical efficiency. Furthermore, there is a need to reduce the complexity of such kits whilst still at least maintaining, preferably enhancing, a surgeon's ability to chose suitable replacement implants.

In a previous patent application, the disclosure of which is incorporated by way of reference, EP2145590A, the applicant has disclosed a kit for secondary knee replacement surgery which obviates many of the disadvantages of previous kits. The adjustable unit includes protruding arms which connects to a bulky external position detector which detects the position of the components *in situ.* This is a somewhat cumbersome arrangement. The trialling of femoral and tibial implants, and the cutting of the femur may also be somewhat inhibited in the kit of that patent application.

The Knee Joint-Line Balancer (apparently described only in the literature in ten Ham, A. M. et al The Knee 12 (2005) 89-92) is an adjustable jig, designed only to work with a Biomet™ knee implant kit for determining the size of trial implants required, indicating the joint line and height of the patella, and allowing better planning of the joint line and patellar position. The jig is fixed to the femur with the knee in flexion, after the cutting of a proximal tibia cut (at 90 degrees to the tibia). After cutting the proximal tibia cut, the size of the femur implant is then estimated on the jig. The knee is then extended and the stability and extension of the knee tested. Finally, the patellar height is measured, and the joint line determined. From the dimensions taken from the jig, suitable implants can be selected. The Knee Joint-Line Balancer only assists in sizing implants, and does not assist a surgeon in cutting the femur or tibia.

It is an object of the invention to provide a simplified yet improved system for knee replacement surgery with aims including reducing the amount of components involved in the surgery, and/or to improve surgical efficiency, and/or to reduce sterilisation costs. In particular, it is an object of the invention to provide a system for knee replacement surgery such as a jig, the use of which leads to improved joint line determination and/or ligament balancing, and to provide a system which is configurable to most closely resemble the final replacement implants, and which has improved stability compared to known systems.

### Summary of the Invention

According to one aspect of the invention there is provide a modular jig for knee replacement surgery for a patient requiring knee replacement surgery, the jig comprising:
a) femoral and tibial size and position-adjustable means operable to replicate the dimensions of suitable femoral and tibial implants, as previously determined by sizing means, and to provide a trial joint;
b) means for adjusting the joint line of the trial joint during trialling of the function of the trial joint;
c) means for adjusting ligament balance of the trial joint; and
d) means for guiding cutting of the femur and or tibia as required to facilitate fitment of final femoral and tibial implants.

According to another aspect of the invention there is provided a sizing, trialling and cutting jig for knee replacement surgery on a patient having a femur and a tibia, the jig comprising:
a) a size adjustable femur joint-specific sizing assembly, which includes a one-piece femoral backplate, the sizing assembly being adjustable on the backplate and functioning as a trial femoral implant, to allow a user to determine dimensions, location and orientation of a suitable replacement femoral implant for the patient and test joint function before providing the patient with a replacement femoral implant,
b) an integral femoral cutting guide, having means for guiding a surgical cutting device to cut the femur,
c) means for adjusting the position of the femur joint-specific sizing assembly in the medial-lateral direction only to adjust femoral offset,
d) a tibial sizing assembly which is mountable on the tibia, and which functions as a trial tibial implant allowing testing of joint function before providing the patient with a replacement tibial implant,
e) a tibia-specific cutting guide having means for guiding a surgical cutting device to cut the tibia,
f) means for adjusting the femoral assembly to adjust ligament balance,
g) the femur joint-specific sizing assembly cooperating with the tibial sizing assembly to replicate the dimensions and operation of a replacement knee joint.

The jig according to either aspect of the present invention has four principle functions i.e. that of determining the correct size implants required, trialling and adjusting the function of the replacement joint, balancing the ligaments, and providing cutting guides for cutting the bone.
According to this aspect of the invention, the adjustable femur joint-specific sizing assembly is adjustable by means of a geared mechanism to move the lowest joint surface away from the longitudinal axis of the femur, increasing the size of the assembly.
The femoral backplate may include a sleeve which receives a stem engaging sleeve of the femoral sizing assembly.
The femoral backplate is preferably of unitary construction, simplifying and strengthening the jig.
The jig may include an additional femoral cutting guide.
The jig may include or cooperate with support means for supporting a patella.

One or each stem for mounting the jig may be provided by a reamer shaft from a reamer with detachable handle or drive means inserted in the femur or tibia.
The tibial joint-specific sizing assembly may include a tibial backplate which has a collar which, in use, is slidably received in a coaxially mounted tubular mount depending from the tibial implant.
A tibia-specific cutting guide may be slidably mounted on an arm extending from and integral with the tibial backplate.
The tibia joint- specific sizing assembly may be fixed to the side of the tibia by fixing means which cooperate with a fixing guide which is slidably mounted on an arm extending from and integral with the tibial backplate.
The femoral joint- specific sizing assembly may be fixed to the side of the femur by fixing means which cooperate with a fixing guide which is slidably mounted on an arm extending from and integral with the femoral backplate.
Suitable fixing means include pins or screws.
Preferably a ruler having visual dimension markings depends downwardly from the tibia joint-specific sizing assembly.More preferably, a ruler extends upwardly from the femoral sizing assembly.
The surgical cutting device is typically a saw.
The invention also provides a tibial joint measuring device, the device comprising an element which fixes to a tibial surface, a plate, overlying the element and the tibia, which is moveable over the element, means for recording the position of the plate in the anterior-posterior and medial-lateral dimensions.
According to another aspect of the invention there is provided a complete surgical tool and component kit for a knee replacement operation in a single instrument box of standard dimensions. The kit of the present invention reduces the number of instrument boxes compared to conventional kits by a factor of 5-10 times. In particular this is made possible because no trial implants are required.
The surgical workflow involved when using a kit in accordance with the invention is significantly shorter than with conventional kits, with the number of surgical manipulations being reduced compared with the use of conventional kits.

The kit may comprise a jig as described above, a tibial joint measuring device as described above, suitable femoral and tibial reamers, femoral and tibial implants According to a further aspect of the invention there is provided a method of knee replacement surgery, the method comprising
a) providing a jig according to the first or second aspects of the invention
b) determining femoral and tibial implant sizes
c) adjusting the position-adjustable means, or femoral and tibial sizing assemblies as appropriate, to replicate the dimensions of suitable femoral and tibial implants, as previously determined, to provide a trial joint;
d) adjusting the joint line if required of the trial joint during trialling of the function of the trial joint;
e) adjusting ligament balance of the trial joint; and
f) cutting of the femur and or tibia, using guides provided by or associated with the jig as required to facilitate fitment of final femoral and tibial implants.

This surgical method is significantly more efficient than previous methods as it does not require fitting and testing numerous trial implants, because the joint function can be readily tested and modified, the bone cuts can be performed using the guides provided with or associated with the jig, and other significant dimensions and orientations can be determined before fitting the final implants.

### Brief Description of the Drawings

A kit, components and tools for secondary knee replacement surgery, and methods of surgery using the kit will now be described, by way of example only, with reference to the accompanying drawings, Figures 1 to 15, in which:
Fig 1 is a schematic perspective view of a sizing and cutting jig in accordance with the invention, mounted on a femur and tibia;
Fig 1A is a schematic perspective view of components of the sizing and cutting jig of Fig 1, showing in particular femoral and tibial rulers mounted on a femoral and tibial components;
Fig 2 is a perspective view of the femoral sizing plate of the jig of Fig. 1;
Fig 2A is a perspective view of the femoral sizing plate of Fig 2 in use;
Fig 3 is a perspective view of a tibial baseplate of a sizing and cutting jig in accordance with the invention;
Fig 4 is a perspective view showing the tibial cutting guide of a sizing and cutting jig in accordance with the invention on a tibia;
Fig 5 is an elevation showing the sizing and cutting jig of Fig 1 on a femur and tibia in flexion;
Fig 6 is a perspective view of a tibial trial implant and tibial backplate;
Fig 7 is a side elevation of the femoral sizing assembly and backplate showing the femoral cutting guides;
Fig 8 is a perspective view of the femoral sizing assembly and backplate of Fig 7 on a femur;
Fig 9 is a perspective view showing an additional femoral cutting guide;
Fig 10 is a side elevation of the additional femoral cutting guide of Fig 9 and a femur;
Fig 11 is a perspective view of the femoral sizing assembly showing an artificial patella and patella support or trochlea;
Fig 12 is a perspective view of a tibial measuring device in accordance with the invention on a tibia;
Fig 13 is a plan view of the tibial measuring device of Fig 12 on a tibia;
Fig 14 is a perspective view of a tibial offset preparation reaming guide in accordance with the invention; and
Fig 15 is a perspective view of a reamer with a detachable handle and shaft for use in a kit and methods of surgery in accordance with the invention.

### Components and tools for knee surgery

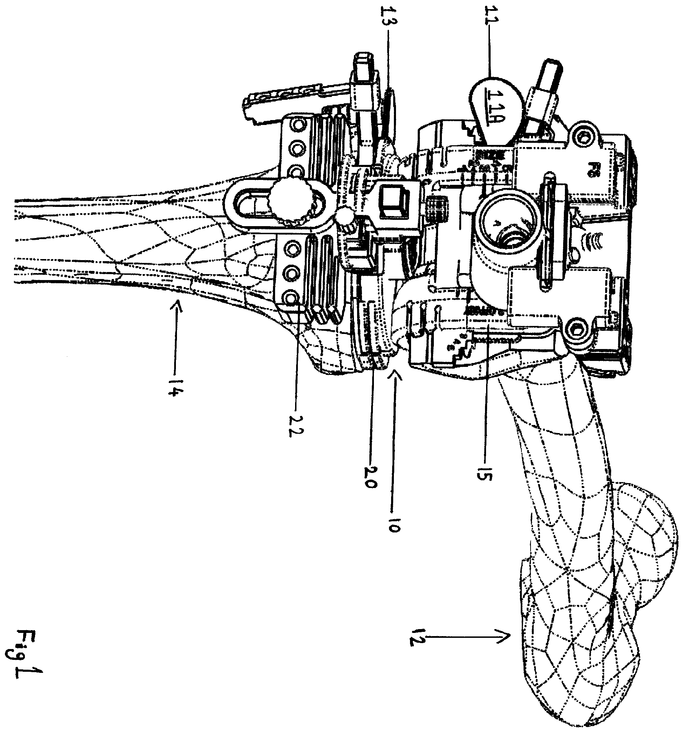

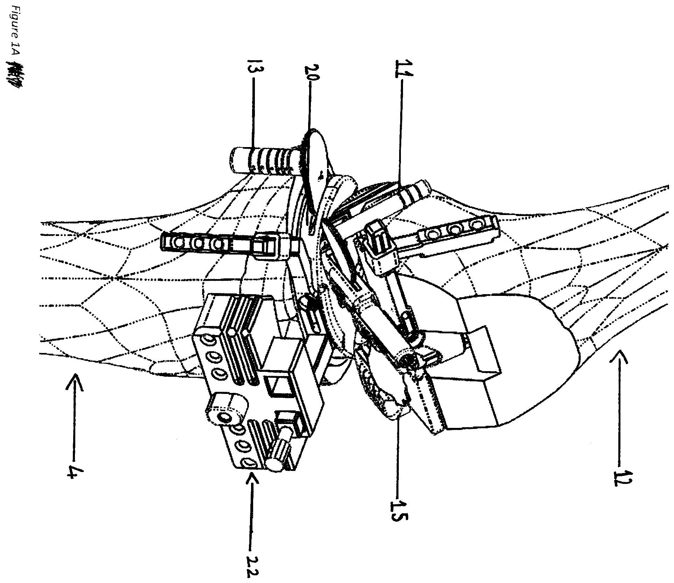

Fig 1 shows a sizing, trialling and cutting jig 10 in accordance with the invention fixed to a left femur 12 and tibia 14 of a patient (not shown) in preparation for a secondary knee replacement, The femur 12 and tibia 14 are shown in flexion. A corresponding jig and other components is provided in a corresponding kit for the right knee. The conventional pins used to fix the jig 10 to the femur 12 and tibia 14 are not shown in Fig 1. and in subsequent drawings. Screws may also be suitable as fixing means as pins.

The jig 10 is relatively compact compared to known jigs and is of modular construction The jig 10 comprises a femur-specific sizing assembly 15 which forms a femoral trial implant, rather than requiring a separate trial femoral implant as used in conventional kits, an integral femur-specific cutting guide 16, a tibial sizing assembly 20 and a tibial cutting guide 22.

As shown particularly in Fig 1A, the jig includes femoral and tibial rulers (11 and 13 respectively) which each have elongate stems with dimension markings thereon, and substantially oval flat bases (e.g. 11A) which are snugly received in lateral slots on the sides of the femur-specific sizing assembly 15 and the tibial sizing assembly 20 so that the rulers are held firmly but so the stems can be moved closer to or away from the relevant bone.

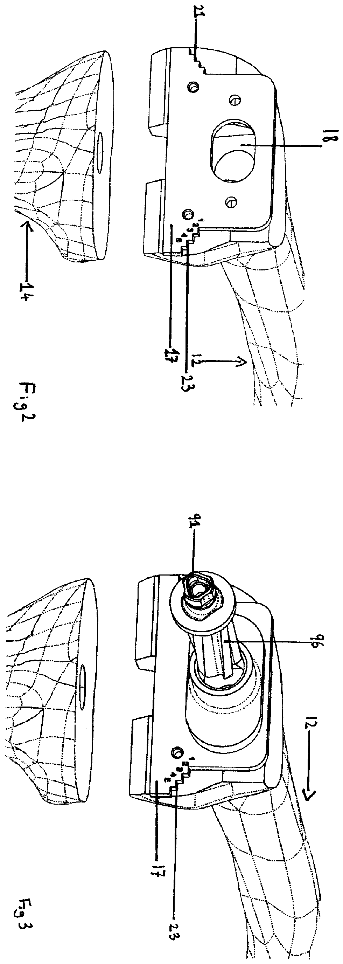

A femoral sizing plate 17, for use before the jig 10, is shown in Fig 2 fixed to the cut lower surface of femur 12 by pins (not shown), driven through pinning holes in the plate 17. The femoral sizing plate 17 has a central elongate aperture 18 and a stepped profile, 21, 23, on either side, representing different femoral implant sizes to be selected. In use, the femoral sizing plate 17 is arranged over the cut lower surface of the femur 12 and the implant sizes marked on the closest "steps" to the opposed edges of the femur 12 (indicated by dotted lines), indicate the final femoral implant size to be used.

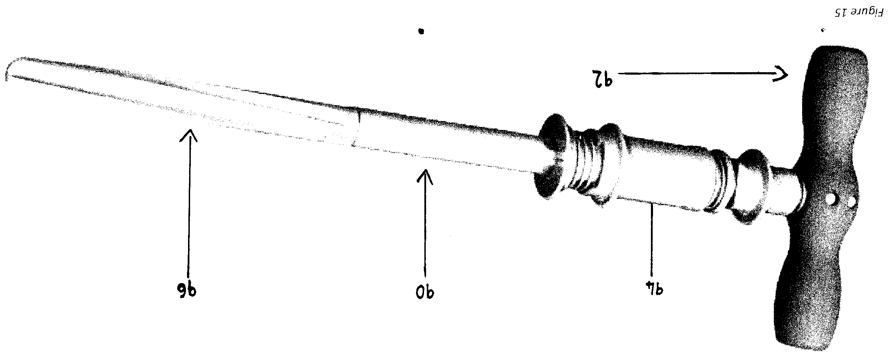

Fig 3 shows the femoral sizing plate 17 in use guiding a reamer 90 (as shown in Fig 15, and of the type with a detachable shaft and handle described in more detail in EP2145590A), of which only the reaming portion 96 is shown, the shaft 94 and handle 92 being omitted for clarity. Fig 3 shows the end 91 of the reaming portion which engages the shaft 94 and after reaming is complete directly engages with an adaptor which in turns connects to the final femoral implant.

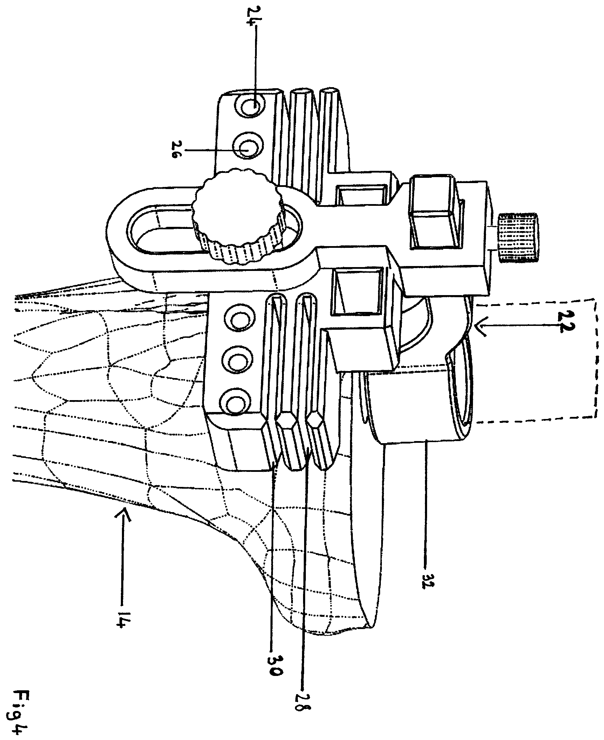

The tibial cutting guide 22 is shown in more detail in Fig 4. The tibial cutting guide 22 is fixed to the tibia 14 by conventional pins inserted through holes (e.g. 24, 26) in the cutting guide's body. The tibia-specific cutting guide's body includes slots 28 and 30 which are sized to receive a surgical saw and arranged to facilitate cutting of the tibia. The tibia specific cutting guide 22 engages with an arm 32A extending from vertically slidable collar 32 which fits about the shaft of a reamer (indicated by the dotted lines in Fig 4.) in a channel reamed in the tibia (not shown) to stabilise the guide 22 when cutting. The collar is adjustable in position in relation to the body of the guide 22. The tibial cutting guide 22 can be readily detached from arm.

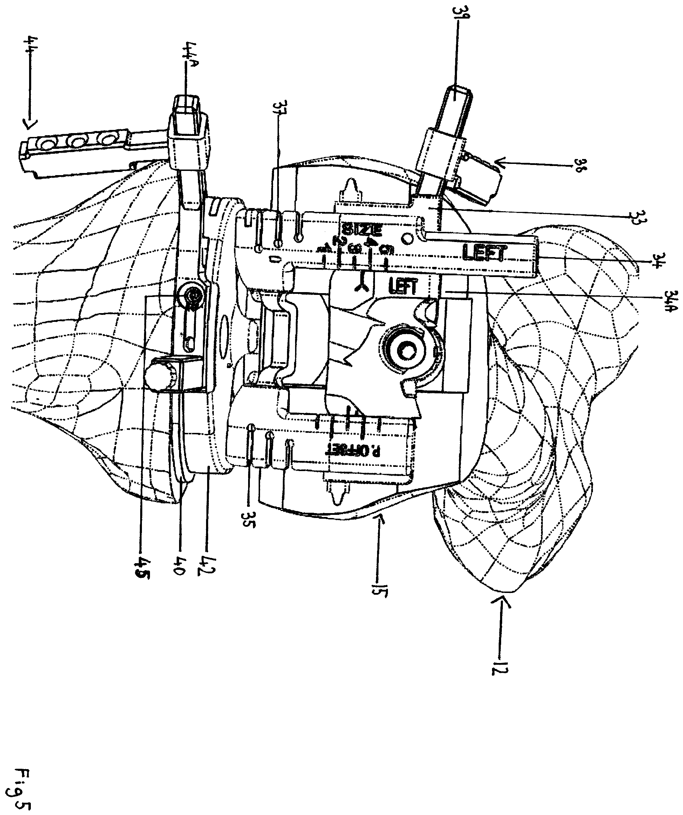

The jig 10's femur-specific cutting and sizing assembly 15, and femur specific cutting guide 18, are shown in more detail in Fig 5. A femoral backplate 33 is fixed directly to the cut lower surface of the femur 12 in the configuration shown in Fig 5. The size of the femoral implant modelled or replicated by the femur-specific cutting and sizing assembly 15 can be increased by operating a screw in a tunnel 34 which causes the assembly 15 to move in the direction of arrow A with respect to the fixed femoral backplate 33. Conversely the operation of the score can be reversed to reduce the size of the femoral implant modelled or replicated by the femur-specific cutting and sizing assembly 15. Posterior offset of the femur-specific cutting and sizing assembly 15 can be altered by operating a screw in a tunnel 34 which causes the assembly 15 anteriorly or posteriorly as required. The markings on the lower surface of the cutting and sizing assembly 15 are shown clearly in Fig 5. The markers represent different sized femoral implants and any offset required in the implant's position. The femoral cutting and sizing assembly 15 is provided with cutting slots e.g. 35, 37 which are parallel with the lower plane of the assembly 15 in the position shown in Fig 5. A slidable femoral pin guide 38 is free to move on arm 39 which is integral with the femoral backplate 33, giving better fixation in practice than with previous jigs, and has holes through which pins can be driven to fix the femur-specific cutting and sizing assembly 15 to the femur 12 during the operation. The arrangement shown also allows easy removal of the assembly 15 , whilst leaving the femoral cutting guide fixed to the femur and femoral backplate 33.

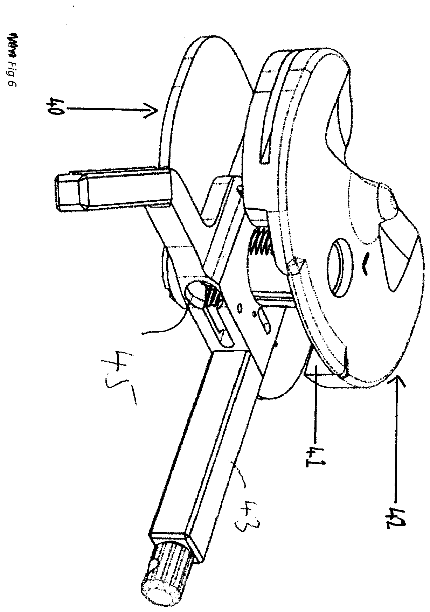

Also shown is a tibial backplate 40, which is fixed to the cut upper surface of the tibia 40, by pins (again not shown) and can be attached to a reamer cutting portion left in the tibia. The tibial backplate 40 is shaped to receive and support a tibial implant 42 (as shown in detail in Fig 6.). Specifically, a tubular portion 44 of the tibial implant 42 is received in a central aperture (obscured) defined by the backplate 40. Rotation of the tibial implant 42 is inhibited by cooperating formations 41, 43 on the respective lower and upper surface of the implant 42 and the backplate 40. The tibial implant 42 can be moveable with respect to the backplate by a screw (hidden) in recess 45, and can be fixed to the tibia 14 by means of pins driven through holes in a slidable tibial pin guide 44 which slides on arm 44A extending from and integral with the backplate. This arrangement gives improved stability.

Only two sizes of tibial and femoral backplate are provided in the kit. Platelet size A is used for implant sizes 1, 2, 3
Platelet size B is used for implant sizes 3, 4, 5. This is another reduction in the number of components required compared to conventional kits.

Fig. 6 shows the flat upper surface of the tibial implant 42 . This flat joint surface provides better stability and reliability during the ligament balancing step.

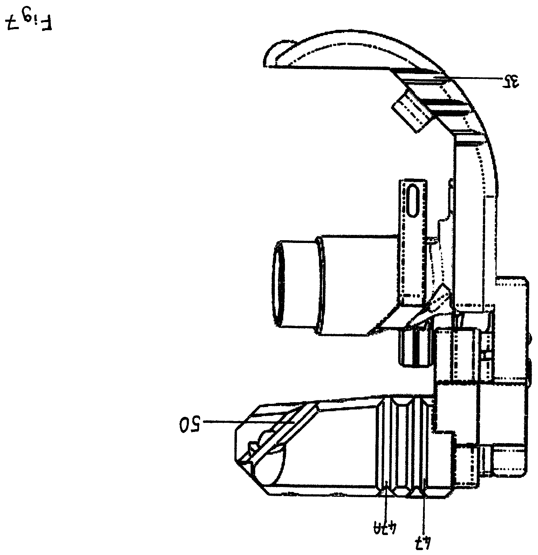

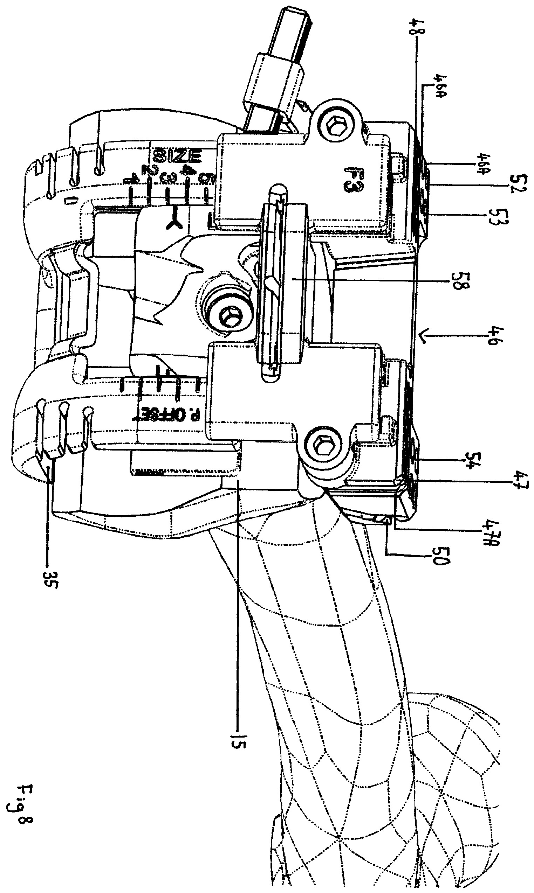

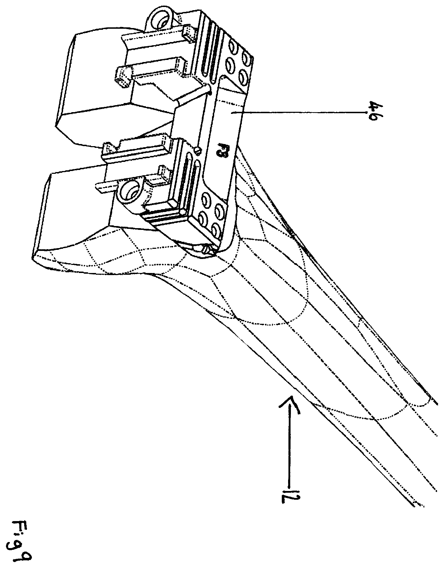

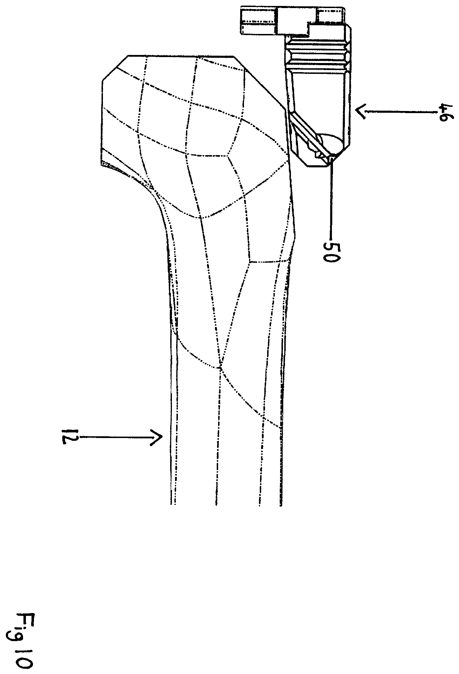

Fig 7, 8, 9 and 10 show in more detail a separate upper femoral cutting guide assembly 46, which is a particular feature of the modular sizing and cutting jig of the invention, and which can be fitted to the upper portion of the femur-specific cutting and sizing assembly 15 as part of jig 10. The separate upper femoral cutting guide 46 includes vertical (in the orientation shown in Fig 7) cutting slots (e.g. 47) which are sized to receive a surgical saw. Corresponding cutting slots (e.g. 48, 48a, 47a) are provided on the other side of the upper femoral cutting guide 46 in the orientation (shown in Fig 7). Separate angled slots 50 and 51 (obscured in Figs 7 and 8) are also provided. The separate upper femoral cutting guide 46 can be fixed to the femur 12 with pins passed through holes (e.g. 52, 54, 56) in the upper surface of the guide. The separate upper femoral cutting guide assembly 46 includes a detachable front portion 58. The main assembly 15 can be removed leaving the separate upper femoral cutting guide 46 in place on the femur, advantageously leaving the surgeon with more room to make the femoral cuts (i.e. femoral augments, anterior cut, chamfers and box cuts) using the guide 46.

Together the tibial cutting assembly, the detachable upper femoral cutting guide assembly 46, and the femoral cutting and sizing assembly 15's cutting slots e.g. 35, 37 provide guides for all major cuts required in secondary knee replacement surgery.

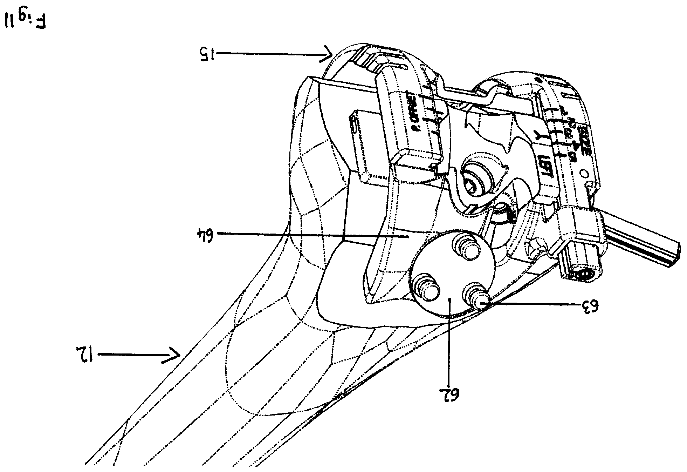

Fig 11 shows a patella support unit 62. The patella support unit 62 includes lugs e.g 63 which a surgeon can mount an original or artificial patella on. The patella support unit 62 is itself held on an artificial trochlea 64 which fixes to the assembly 15, allowing the surgeon to model the patellar path with the final implants.

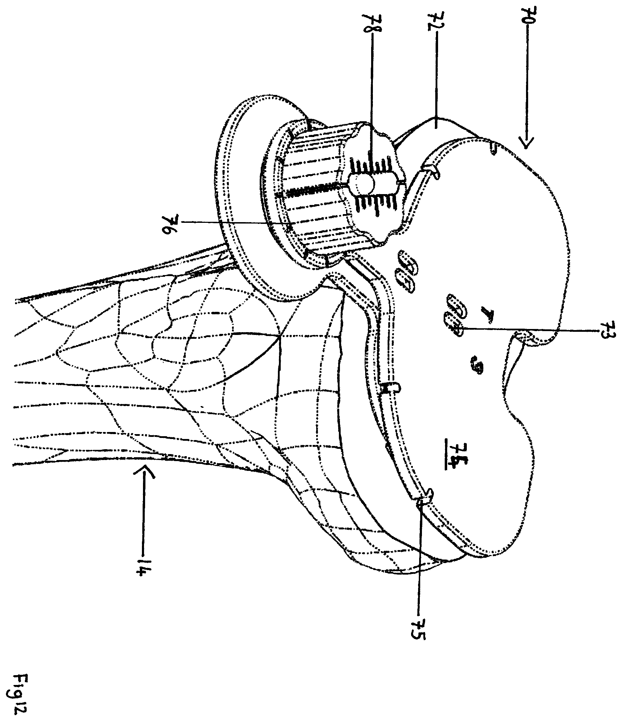

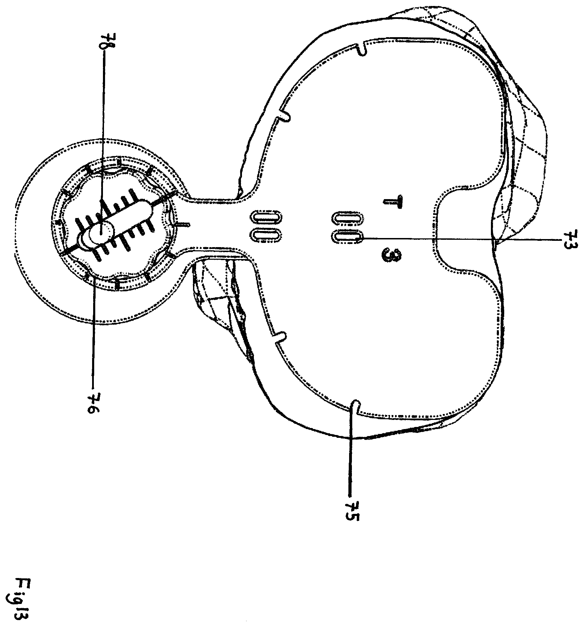

Fig 12 and 13 show a tibial measuring device 70 of the invention, arranged on a cut upper surface 71 of a tibia 14, for determining the size of tibial implant - and therefore tibial backplate, and the amount of any offset, required prior to use of jig 10. The tibial measuring device 70 comprises a flat base 72, and a flat tibial implant size-specific plate 74 which, overlies the base. The plate 74 shown in Figs 12 and 13 corresponds to a T3 size tibial implant, and has four pin holes (e.g. 73) and four peripheral notches (e.g. 75), which are sized to allow a surgeon to mark the bone below with a bistoury to record the position of the plate 74, this indicating the optimal position of the tibial implant. A measuring knob 76, and a sliding indicator 78, are connected to the plate 74 so that rotation of the knob 76 causes lateral and longitudinal movement of the plate 74, allowing the surgeon to move the plate 74 to optimally overlie the upper surface 71, of the tibia 14. The degree of rotation of the knob 76 and linear movement of the indicator 78 are indicated by markings on adjacent surfaces around the knob 76 and indicator 78. In use, the knob 76 is used to move the tibial implant size-specific plate 74 over the base 72 so that the plate 74 is optimally positioned over the cut upper surface of tibia 14. The position of the plate 74 is then recorded by the surgeon by marking the bone below with a manual or electric bistoury.

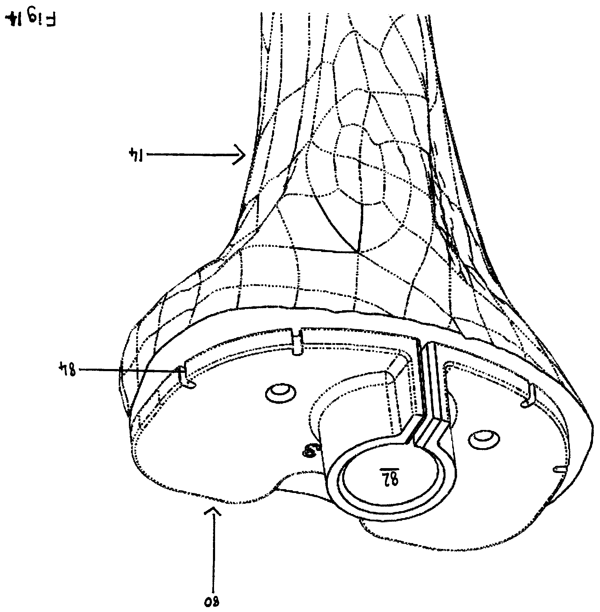

Fig 14 shows a tibial offset preparation reaming guide 80. The guide 80 has a central channel 82 to receive in use a reamer (such as reamer 90 described below). The guide 80 is sized to match tibial implant size-specific plate 74 and the periphery of the guide 80 also defines notches (e.g. 84) which match the size and position of the peripheral notches (e.g. 75) of the plate 74. The guide is arranged in use so that its notches overly the marks made on the tibia by the surgeon as described above.

Fig 15 shows a manual reamer 90 for use with the kit of the invention. The reamer 90 is as shown in the applicant's earlier European patent application, comprising a detachable handle 92 and shaft 94, and reaming portion 96. An improved connection between the shaft 94 and reaming portion 96 means that motorised reamers can also be used with the kit of the invention, reducing the time taken for surgery and therefore improving process efficiency, reducing costs and patient risk.

A kit for replacement knee surgery is constituted by as core core components: a jig 10: a two femoral backplates 33 of different sizes and two tibial backplates 40; two reamers, femoral and tibial reaming guides, a femoral sizing plate 17, a tibial measuring device 70, a selection of suitable final femoral and tibial implants and adaptors for connecting the stems. The kit can be contained in suitable a standard instrument box (either 518x244x114 (mm); 518x244x76 (mm); or 230x244x76 (mm) for storage, handling and sterilising.

### Methods of surgery

Methods of secondary knee replacement surgery using the previously described components of the kit of the invention will now be described, by way of example only, using the same reference numbers to denote features as before.

The surgery is performed on a patient who has previously had a knee replacement. Accordingly the lower end of the femur has been shaped during previous surgery leaving a flat lower surface and chamfered ends to the femur. The tibia has also been cut at its upper end to form a plateau, but there may have been degradation of this plateau.

### Femoral reaming, Femoral sizing, and offset measure

In a first step, a central channel is reamed out in the femur using the reamer of Fig 15 or a motorised reamer. After reaming out the channel, the reamer handle 92 is removed and a femoral sizing plate 17 as shown in Fig 2 is slid down the shaft 94 of the reamer towards the the cut lower surface of femur 12. Using the marks on the femoral signing plate 17 the degree of femoral offset can be measured. Using the stepped profile 21, 23 on either side of plate 17 the femoral implant size can be determined, which indicates which size of femoral implant will be required - and correspondingly which sized tibial implant is needed. If an offset is needed, the femoral baseplate is pinned to the lower surface of the femur in a 4mm (either laterally or medially) offset position, and an offset reaming guide 80 is fitted to overlie the outer surface of the femoral sizing plate 17. A reamer is then inserted through the central aperture of the offset reaming guide and an offset channel reamed in the femur 12.

### Tibial Reaming

In a second reaming step, a channel is reamed in the tibia, using a different reamer. The handle and of the reamer is then removed leaving the reaming portion and shaft of the reamer *in situ.*

The tibial cutting guide 22 is then fixed to the tibia 14 by conventional pins inserted through holes (e.g. 24, 26) in the cutting guide body. The collar 32 of the cutting guide 22 is fitted about the shaft of the reamer in a channel reamed in the tibia 14 to stabilise the guide when cutting. The slots (e.g. 28, 30) of the tibia-specific cutting guide are used to guide a surgical saw to make a flat reference, 4° slope tibial plane, thus creating a fresh tibial plateau.

### Selecting Femoral and Tibial Backplates

Having determined the appropriate sizes of femoral and tibial implants, the femur-specific sizing assembly 15 which replicates or forms a femoral trial implant is fitted to an appropriately sized i.e having the previously determined size, femoral backplate 33.

Similarly, an appropriately sized tibial implant is selected from the kit and fitted to the tibial backplate.

Both the femoral and tibial components are in turn mounted over reamer reaming portions 96 left in channels as described above.

This results in the femoral and tibial components being connected to form an artificial knee joint provided by the jig 10.

### Medial/Lateral Femoral Offset

The position of the femoral sizing assembly 15 can be adjusted sideways in the medial-lateral directions only to allow for femoral offset. In practice, this functionality simplifies the components of the cutting and sizing jig enhancing surgical efficiency.

The femoral sizing assembly is then expanded to the previously determined femoral implant size, by operation of the screw at 34 until the markings shown in particular in Fig 5 align with the determined femoral implant size.

### Fixation of the femoral and tibial components

The jig 10 is further fixed to the femur and tibia by pinning through the pin guides 38,44 which are slide on arms extending from the respective backplates.

### Mounting the trochlea and patella support

The patella support unit 62 is mounted on an artificial trochlea 64 fixed to the femoral assembly 15, allowing the surgeon to model the patella's path with the final implants throughout the joint line determination and balancing.

### Joint line determination

The surgeon then mounts the femoral and tibial rulers 11,13 in place on the femoral and tibial sizing assemblies and rotates their oval bases to set the stems at a correct distance from the femur and tibia respectively. The femoral ruler can be adjusted to verify position of the femoral insertion of the medial collateral ligament. The screw at 34 can be operated to extend or withdraw the femoral sizing assembly 15 to adjust the position of the joint line as required to optimise joint line location.

### Ligament Balancing

The tibial implant 42 can be moved up or down by operation of a screw 45, and the joint manipulated by the surgeon to adjust the ligament balance. The height required for the tibial implant can be determined by use of the tibial ruler 13, the lower plane of the arm 44A corresponding to the plane of the tibial plateau.

Posterior offset can also be determined at this stage, adjusting the screw on the assembly 15 as required.

### Posterior augment cuts

Posterior augment cuts are then made in the femur as required using the guide slots e.g. 35 in the femoral sizing assembly 15.

### Further femoral cuts

The additional separable upper femoral cutting guide assembly 46, which is a feature of the modular sizing and cutting jig of the invention, is fitted to the upper portion of the femur-specific cutting and sizing assembly 15 and pinned with parallel pins to the femur 12. An anterior chamfer cut can then be made using the guides e.g 50. The assembly can then be pinned with oblique pins and any augment cuts made.

The detachable front portion 58 is then removed along with the remainder of the femoral sizing assembly, leaving just the upper femoral cutting guide assembly 46 pinned to the femur. Any box cuts can be made easily by the surgeon, the modularity of the jig allowing only the necessary separable upper femoral cutting guide assembly 46 in place pinned to the femur, thus maximising the surgeon's freedom to operate.

### Tibial Augment Cuts

The tibial cutting guide 22 is held in relation to the tibia by the arm 43 extending from the tibial backplate (for example in the configuration shown in Fig 1).

Using the posterior visualiser to determine the level of augment needed and the guide is pinned to the bone. The surgeon then cuts using the appropriate slot, either leaving the jig 10 in place, or removing it by first.

### Tibial offset measure

To determine any tibial offset required the surgeon connects a reamer to the tibial measuring device 70 , and places it on top of the tibial plane. The device is operated to slide the plate 74 over the base 72 until a satisfactory coverage of the tibia is achieved.

The surgeon reads the offset length and the angle on the markings 78 and 76. The surgeon marks the position of the base 72 using a bistoury in the four peripheral notches before removing the device 70.

If an offset is needed, the surgeon aligns the tibial offset reaming guide 80 so that its peripheral notches align with the marks made above, pins the guide 80 to the bone and deepens the reaming done in the previous steps as required for component fit.

Lodges can be cut at this time in the tibial surface to provide room for any tibial keel

### Fitting final implants

Final femoral and tibial implants can be fitted with any final augments as required. The resulting (augmented) implants are then mounted on neutral (i.e. coaxial with the stem) or offset adaptors as required fixed to the femoral and tibial stems provided by the reaming positions left *in situ* in the femur and tibia respectively. The stems may be cemented. The femoral and tibial constructs may be hammered in place.

The embodiments of the invention described in this patent specification may be altered within the scope or spirit of the invention, for example by replacing components with technical or functional equivalents. The use of reference numerals in the specification and claims is for ease of intelligibility only and does not limit the scope of the claims in any way.

## Claims

1. A modular jig 10 for knee replacement surgery for a patient requiring knee replacement surgery, the jig 10 comprising:
a) femoral and tibial size and position-adjustable means 15, 20 operable to replicate the dimensions of suitable femoral and tibial implants, as previously determined by sizing means, and to provide a trial joint;
b) means for adjusting the joint line of the trial joint during trialling of the function of the trial joint;
c) means for adjusting ligament balance of the trial joint; and
d) means for guiding cutting of the femur and or tibia as required to facilitate fitment of final femoral and tibial implants.

2. A sizing, trialling and cutting jig 10 for knee replacement surgery on a patient having a femur and a tibia, the jig 10 comprising:
a) a size adjustable femur joint-specific sizing assembly 15, which includes a one-piece femoral backplate 33, the sizing assembly 15 being adjustable on the backplate 33, and functioning as a trial femoral implant, to allow a user to determine dimensions, location and orientation of a suitable replacement femoral implant for the patient and test joint function before providing the patient with a replacement femoral implant,
b) an integral femoral cutting guide, having means for guiding a surgical cutting device to cut the femur,
c) means for adjusting the position of the femur joint-specific sizing assembly 15 in the medial-lateral direction only to adjust femoral offset,
d) a tibial sizing assembly 20,40 which is mountable on the tibia, and which functions as a trial tibial implant allowing testing of joint function before providing the patient with a replacement tibial implant,
e) and a tibia-specific cutting guide 22 having means for guiding a surgical cutting device to cut the tibia,
f) means for adjusting the femoral assembly 15 to adjust ligament balance,
g) the femur joint-specific sizing assembly 15 cooperating with the tibial sizing assembly 40 to replicate the dimensions and operation of a replacement knee joint.

3. A jig according to claim 2 in which the adjustable femur joint-specific sizing assembly is adjustable by means of a geared mechanism to move the lowest joint surface away from the longitudinal axis of the femur, increasing the size of the assembly.

4. A jig according to claim 2 or 3 in which the femoral backplate includes a sleeve which receives a stem adaptor engaging sleeve of the femoral sizing assembly 15.

5. A jig according to any one of claims 2 to 4 in which the femoral backplate is of unitary construction.

6. A jig according to any one of claims 2 to 5 which is adjustable to balance the ligaments.

7. A jig according to any one of claims 2 to 6 including an additional femoral cutting guide.

8. A jig according to any preceding claim comprising support means for supporting a patella.

9. A jig according to claim 8 including an artificial patella mounted on the support means.

10. A jig according to any preceding claim in which one or each stem for mounting the jig is provided by a reamer shaft from a reamer with detachable handle or drive means inserted in the femur or tibia.

11. A jig according to any one of claims 2 to 10 in which the tibial joint-specific sizing assembly includes a tibial backplate which has a collar which, in use, is slidably received in a coaxially mounted tubular mount depending from the tibial implant.

12. A jig according to any one of claims 2 to 11 in which the tibia-specific cutting guide is slidably mounted on an arm extending from and integral with the tibial backplate.

13. A jig according to any one of claims 2 to 12 in which the tibia joint- specific sizing assembly can be fixed to the side of the tibia by fixing means which cooperate with a fixing guide which is slidably mounted on an arm extending from and integral with the tibial backplate.

14. A jig according to any one of claims 2 to 13 in which the femoral joint- specific sizing assembly can be fixed to the side of the femur by fixing means which cooperate with a fixing guide which is slidably mounted on an arm extending from and integral with the femoral backplate.

15. A sizing and cutting jig according to claim 13 or 14 in which the the fixing means are pins or screws.

16. A jig according to any one of claims 2 to 15 in which a ruler having visual dimension markings depends downwardly from the tibia joint-specific sizing assembly.

17. A jig according to any one of claim 2 to 16 in which a ruler extends upwardly from the femoral sizing assembly.

18. A jig according to any preceding claim in which the surgical cutting device is a saw

19. A tibial joint measuring device, the device comprising an element which fixes to a tibial surface, a plate, overlying the element and the tibia, which is moveable over the element, means for recording the position of the plate in the anterior-posterior and medial-lateral dimensions.

20. A complete surgical tool and component kit for a knee replacement operation in a single instrument box of standard dimensions.

21. A kit according to claim 20, the kit comprising a jig according to any one of claims 1 to 19, a tibial joint measuring device according to claim 19, femoral and tibial reamers, femoral and tibial implants

22. A method of knee replacement surgery, the method comprising
a) providing a jig according to any one of claims 1 to 18
b) determining femoral and tibial implant sizes
c) adjusting the position-adjustable means 15, 20 to replicate the dimensions of suitable femoral and tibial implants, as previously determined, to provide a trial joint;
d) adjusting the joint line if required of the trial joint during trialling of the function of the trial joint;
e) adjusting ligament balance of the trial joint; and
f) cutting of the femur and or tibia, using guides provided by or associated with the jig as required to facilitate fitment of final femoral and tibial implants.
